# EUROPÄISCHE PATENTSCHRIFT

(11) **EP 3 347 690 B1**
(45) Veröffentlichungstag und Bekanntmachung des Hinweises auf die Patenterteilung: **06.10.2021**
(21) Anmeldenummer: 16741845.8
(22) Anmeldetag: 11.07.2016
(51) Int. Cl.: G01N 11/08

(54) **MESSDÜSE ZUR BESTIMMUNG DER DEHNVISKOSITÄT VON KUNSTSTOFFSCHMELZEN**
MEASURING NOZZLE FOR DETERMINING THE EXTENSIONAL VISCOSITY OF PLASTIC MELTS
BUSE DE MESURE DESTINÉE À LA DÉTERMINATION DE LA VISCOSITÉ ÉLONGATIONNELLE DE MATIÈRES PLASTIQUES EN FUSION

(30) Priorität: 22.07.2015 AT 506442015
(43) Veröffentlichungstag der Anmeldung: 18.07.2018
(73) Patentinhaber: Leistritz Extrusionstechnik GmbH, 90459 Nürnberg (DE)
(72) Erfinder: KÖPPLMAYR, Thomas, 4020 Linz (AT)
(74) Vertreter: Lindner Blaumeier Patent- und Rechtsanwälte
(86) Internationale Anmeldenummer: PCT/AT2016/050247
(87) Internationale Veröffentlichungsnummer: WO 2017/011844

(56) Entgegenhaltungen:
- THOMAS J. OBER ET AL: "Microfluidic extensional rheometry using a hyperbolic contraction geometry", RHEOLOGICA ACTA., Bd. 52, Nr. 6, 1. Juni 2013 (2013-06-01), Seiten 529-546, XP055299647, DE ISSN: 0035-4511, DOI: 10.1007/s00397-013-0701-y
- D.R. OLIVER ET AL: "The flow of polymer thickened motor oils in convergent jet thrust nozzles", JOURNAL OF NON-NEWTONIAN FLUID MECHANICS., Bd. 2, Nr. 4, 1. Juli 1977 (1977-07-01), Seiten 367-384, XP055309396, NL ISSN: 0377-0257, DOI: 10.1016/0377-0257(77)80022-0

## Beschreibung

### Technisches Gebiet

Die Erfindung bezieht sich auf eine Messdüse zur Bestimmung der Dehnviskosität von Kunststoffschmelzen während ihrer Verarbeitung mit einem einen rechteckigen Querschnitt aufweisenden Strömungskanal, der zwischen einem Einlaufabschnitt konstanten Querschnitts und einem Auslaufabschnitt konstanten Querschnitts einen Übergangsabschnitt aufweist, dessen Breite sich in Strömungsrichtung hyperbolisch verjüngt.

### Stand der Technik

Eine Bestimmung der Dehnviskosität mithilfe von Druckfühlern, die einer Verjüngung eines Strömungskanals einer Messdüse vor- und nachgeordnet sind, setzt eine konstante mittlere Dehnrate der Polymerschmelze im Verjüngungsabschnitt des Strömungskanals voraus. Zu diesem Zweck ist es bekannt (US 5 357 784 A, US 6 220 083 B1), eine Messdüse mit einem Einlaufabschnitt und einem Auslaufabschnitt jeweils konstanten Querschnitts und mit einem den Einlaufquerschnitt auf den Auslaufquerschnitt verjüngenden Übergangsabschnitt vorzusehen, der zur Ausbildung der Verjüngung zwei einander gegenüberliegende Kanalwände mit einem hyperbolischen Verlauf, im Übrigen aber einen rechteckigen Querschnitt zwischen den konvergierenden Kanalwänden und den diese konvergierenden Kanalwände miteinander verbindenden, zueinander parallel verlaufenden Kanalwänden aufweist. Bedingt durch diese Geometrie des Übergangsabschnitts zwischen dem Einlauf- und dem Auslaufabschnitt der Messdüse kann eine weitgehend konstante mittlere Dehnungsrate für die Polymerschmelze sichergestellt werden, allerdings mit dem Nachteil eines vergleichsweise geringen Druckabfalls, was bei höheren Anforderungen an die Messgenauigkeit eine hohe Ansprechempfindlichkeit der eingesetzten Druckfühler erfordert. Diesen Anforderungen können handelsübliche Druckfühler kaum genügen. Außerdem können solche Druckfühler wegen der Verjüngung des Strömungskanals nicht unmittelbar an den Auslaufabschnitt angeschlossen werden, sodass zum Anschluss der Druckfühler Messkapillaren gebohrt werden müssen, in denen sich Polymerschmelze anlagert, wodurch es nicht nur zu einer Schädigung der Polymerschmelze, sondern auch zu einer Verfälschung des Messergebnisses durch eine teilweise Verlegung der Messkapillaren kommen kann.

OBER ET AL: "Microfluidic extensional rheometry using a hyperbolic contraction geometry", RHEOLOGICA ACTA., Bd. 52, Nr. 6, 1. Juni 2013 (2013-06-01), Seiten 529-546, ISSN: 0035-4511, DOI: 10.1007/s00397-013-0701-y, offenbart eine Messdüse zur Bestimmung der Dehnviskosität von Kunststoffschmelzen während ihrer Verarbeitung mit einem einen rechteckigen Querschnitt aufweisenden Strömungskanal, der zwischen einem Einlaufabschnitt und einem Auslaufabschnitt jeweils konstanten Querschnitts einen Übergangsabschnitt aufweist, dessen Breite sich in Strömungsrichtung hyperbolisch verjüngt, wobei sich die Breite des Übergangsabschnitts im Anschluss an die Verjüngung hyperbolisch auf die der Breite des Einlaufabschnitts entsprechende Breite des Auslaufabschnitts erweitert.

### Darstellung der Erfindung

Der Erfindung liegt somit die Aufgabe zugrunde, eine Messdüse zur Bestimmung der Dehnviskosität von Polymerschmelzen so auszubilden, dass nicht nur handelsübliche Druckfühler eingesetzt werden können, sondern auch eine ausreichende Messgenauigkeit gewährleistet werden kann.

Ausgehend von einer Messdüse der eingangs geschilderten Art löst die Erfindung die gestellte Aufgabe dadurch, dass, zusätzlich dazu, dass sich die Breite des Übergangsabschnitts im Anschluss an die Verjüngung hyperbolisch auf die der Breite des Einlaufabschnitts entsprechende Breite des Auslaufabschnitts erweitert, sich auch die Höhe des Übergangsabschnitts stetig von der Höhe des Einlaufabschnitts auf die kleinere Höhe des Auslaufabschnitts verringert.

Durch die hyperbolische Erweiterung des Strömungskanals im Anschluss an die hyperbolische Verjüngung wird in Verbindung mit der sich in Strömungsrichtung stetig verringernden Höhe des Übergangsabschnitts eine Verlängerung des Strömungsabschnitts ermöglicht, in dem eine konstante mittlere Dehnrate vorherrscht. Es muss ja lediglich der Höhenverlauf und der hyperbolische Wandverlauf aufgrund bekannter Zusammenhänge so aufeinander abgestimmt werden, dass sich eine nach einer hyperbolischen Funktion in Strömungsrichtung ändernde Querschnittsfläche des Strömungskanals zur Aufrechterhaltung einer konstanten mittleren Dehnungsrate ergibt. Diese Geometrie führt zu einer Vergrößerung des Druckverlusts, auf dessen Basis die Dehnviskosität berechnet wird. Damit werden die konstruktiven Voraussetzungen zum Einsatz handelsüblicher Druckfühler geschaffen, die trotz einer mäßigen Ansprechempfindlichkeit in Verbindung mit der erfindungsgemäßen Messdüse nicht nur höhere Anforderungen an die Messgenauigkeiten erfüllen, sondern auch unmittelbar an die Ein- und Auslaufabschnitte der Messdüse angeschlossen werden können, womit die durch Messkapillaren bedingten Nachteile entfallen. Außerdem können in einfacher Weise höhere Aspektverhältnisse zwischen der Breite und der Höhe des Strömungsquerschnitts eingehalten werden, was der Forderung nach einem diesbezüglichen Aspektverhältnis von mindestens 10 zugutekommt, das für die der Bestimmung der Dehnviskosität zugrundeliegenden Formeln vorausgesetzt wird.

Um insbesondere handelsübliche Druckfühler einfach an die Messdüse anschließen zu können, kann der Strömungskanal auf seiner Breitseite sowohl im Einlaufabschnitt als auch im Auslaufabschnitt wenigstens eine Anschlussöffnung für einen Druckfühler aufweisen, sodass sonst unter Umständen erforderliche Messkapillaren entfallen.

### Kurze Beschreibung der Zeichnungen

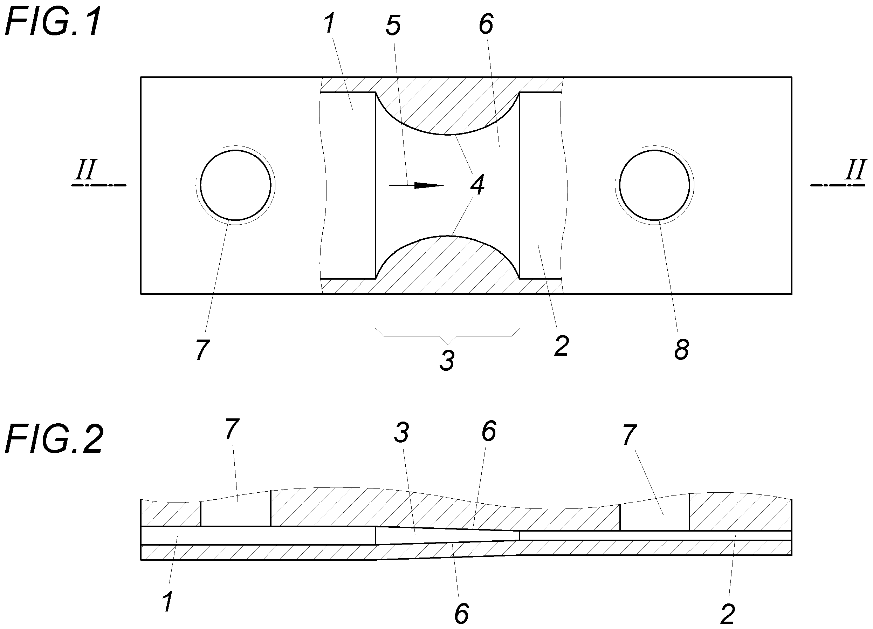

In der Zeichnung ist der Erfindungsgegenstand beispielsweise dargestellt. Es zeigen
- Fig. 1: eine erfindungsgemäße Messdüse in einer schematischen, zum Teil aufgerissenen Draufsicht und
- Fig. 2: diese Messdüse im Längsschnitt nach der Linie II-II der Fig. 1.

### Weg zur Ausführung der Erfindung

Die Messdüse gemäß dem dargestellten Ausführungsbeispiel weist einen beispielsweise an einen Extruder anschließbaren Einlaufabschnitt 1 und einen Auslaufabschnitt 2 mit jeweils übereinstimmenden, konstanten, rechteckigen Querschnitten auf. Zwischen dem Einlauf- und Auslaufabschnitt 1, 2 ist ein Übergangsabschnitt 3 vorgesehen, in dem die die Breite des Strömungskanals begrenzenden Kanalwände 4 zunächst in Strömungsrichtung 5 nach einer hyperbolischen Funktion konvergieren und im Anschluss daran wieder hyperbolisch divergieren, sodass ein- und auslaufseitig des Übergangsquerschnitts 3 der Strömungskanal eine übereinstimmende Breite aufweist. Die die Höhe des Strömungskanals begrenzenden Kanalwände 6 weisen einen sich in Strömungsrichtung 5 stetig verringernden Abstand auf, wobei die Höhe der Strömungsquerschnitte im Bereich des Übergangsabschnitts 3 auf den hyperbolischen Verlauf der Kanalwände 4 derart abgestimmt ist, dass sich eine über die Länge des Übergangsabschnitts 3 konstante, mittlere Dehnungsrate für die die Messdüse durchströmende Polymerschmelze einstellt.

Aufgrund der besonderen Geometrie der erfindungsgemäßen Messdüse wird der Übergangsbereich 3, in dem eine konstante mittlere Dehnungsrate auftritt, gegenüber herkömmlichen Messdüsen mit einem sich lediglich hyperbolisch verjüngenden Strömungskanalabschnitt deutlich verlängert, was zu einem größeren Druckabbau und damit zu einer Erhöhung der Messempfindlichkeit führt, sodass auch mit handelsüblichen Druckfühlern gute Messergebnisse erzielt werden können. Die wegen der besonderen Gestaltung des Übergangsabschnitts 3 gleiche Breite des Einlauf- und Auslaufabschnitts 1, 2 bietet außerdem eine vorteilhafte Voraussetzung dafür, die Druckfühler im Bereich des Einlauf- und Auslaufabschnitts 1, 2 unter Vermeidung von Messkapillaren unmittelbar an den Strömungskanal anzuschließen. Dies wird in dem Ausführungsbeispiel durch entsprechende Anschlussöffnungen 7 für handelsübliche Druckfühler angedeutet.

## Patentansprüche

1. Messdüse zur Bestimmung der Dehnviskosität von Kunststoffschmelzen während ihrer Verarbeitung mit einem einen rechteckigen Querschnitt aufweisenden Strömungskanal, der zwischen einem Einlaufabschnitt (1) und einem Auslaufabschnitt (2) jeweils konstanten Querschnitts einen Übergangsabschnitt (3) aufweist, dessen Breite sich in Strömungsrichtung hyperbolisch verjüngt, wobei sich die Breite des Übergangsabschnitts (3) im Anschluss an die Verjüngung hyperbolisch auf die der Breite des Einlaufabschnitts (1) entsprechende Breite des Auslaufabschnitts (2) erweitert, **dadurch gekennzeichnet, dass** sich die Höhe des Übergangsabschnitts (3) stetig von der Höhe des Einlaufabschnitts (1) auf die kleinere Höhe des Auslaufabschnitts (2) verringert.

2. Messdüse nach Anspruch 1, **dadurch gekennzeichnet, dass** der Strömungskanal auf seiner Breitseite sowohl im Einlaufabschnitt (1) als auch im Auslaufabschnitt (2) wenigstens eine Anschlussöffnung (7) für einen Druckfühler aufweist.

## Claims

1. Measuring nozzle for determining the extensional viscosity of plastic melts during the processing thereof, comprising a flow channel which has a rectangular cross section and which, between an inlet portion (1) and an outlet portion (2) of in each case constant cross section, has a transition portion (3), the width of which tapers hyperbolically in the flow direction, wherein the width of the transition portion (3), following the taper, widens hyperbolically to the width of the outlet portion (2), which corresponds to the width of the inlet portion (1), **characterized in that** the height of the transition portion (3) decreases continuously from the height of the inlet portion (1) to the lesser height of the outlet portion (2).

2. Measuring nozzle according to Claim 1, **characterized in that** the flow channel has on its broad side, both in the inlet portion (1) and in the outlet portion (2), at least one attachment opening (7) for a pressure sensor.

## Revendications

1. Buse de mesure destinée à déterminer la viscosité d'extension de matières synthétiques fondues pendant leur traitement, ladite buse comportant un canal d'écoulement à section transversale rectangulaire qui comporte entre une portion d'entrée (1) et une portion de sortie (2), les deux portions ayant section constante, une portion de transition (3) dont la largeur se rétrécit hyperboliquement dans le sens de l'écoulement, la largeur de la portion de transition (3) s'élargissant hyperboliquement à la suite du rétrécissement jusqu'à la largeur de la portion de sortie (2) qui correspond à la largeur de la portion d'entrée (1), **caractérisée en ce que** la hauteur de la portion de transition (3) diminue continument de la hauteur de la portion d'entrée (1) à la plus petite hauteur de la portion de sortie (2).

2. Buse de mesure selon la revendication 1, **caractérisée en ce que** le canal d'écoulement comporte au moins un orifice de raccordement (7) destiné à un capteur de pression sur son côté large aussi bien dans la portion d'entrée (1) que dans la portion de sortie (2) .
